# EUROPEAN PATENT APPLICATION

(11) **EP 2 491 855 A1**
(43) Date of publication of application: **29.08.2012**
(21) Application number: 11155617.1
(22) Date of filing: 23.02.2011
(51) Int. Cl.: A61B 5/00, A61B 5/145

(54) **Biomedical device capable of using an earphone and microphone plug to transmit data and method for transmitting data**

(71) Applicant: Pensiero Medical Electronics Corp., Fude 1st Road Xizhi District New Taipei City (CN)
(72) Inventor: Chen, Chun-Yu, Zhonghe City (TW); Yang, Chia-Wen, Zhonghe City (TW); Liao, Wei-Cheng, Zhonghe City (TW); Chen, Hsuan-Che, Zhonghe City (TW); Chen, Yen-Hsiung, Zhonghe City (TW)
(74) Representative: Becker Kurig Straus

(57) **Abstract**

The present invention relates to a biomedical device capable of using an earphone and microphone plug to transmit data and a method for transmitting data, wherein the biomedical device comprises: a measuring unit, a micro-control unit, an earphone and microphone plug, a switch unit, a level shift unit, an amplifying unit, and a power management unit. By way of inserting the earphone and microphone plug into an earphone and microphone jack of a portable electronic device, the biomedical device is able to transmit data to the portable electronic device without passing the certification of the transmission format defined by a potable electronic device vender in advance. Moreover, through the method, a user can input biomedical data into the portable electronic device, so as to record and trace the daily biomedical data thereof; in addition, the biomedical data can be uploaded to a Cloud Database via the portable electronic device for a telemedicine management.

## Description

### BACKGROUND OF THE INVENTION

### 1. Technical Field

The present invention relates to a biomedical device, and more particularly, to a biomedical device capable of using an earphone and microphone plug to transmit data and a method for transmitting data, in which the biomedical device is able to communicate with a portable electronic device by way of inserting the earphone and microphone plug into an earphone and microphone jack of the portable electronic device.

### 2. Description of Related Art

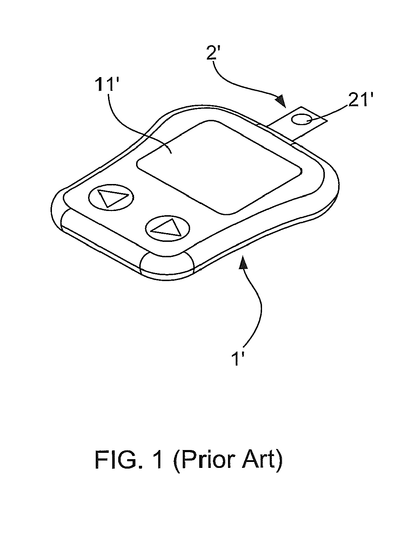

Recently, various biomedical devices are widely used in human life, such as the blood glucose meter, the body fat meter, the blood pressure meter, the electrocardiography meter, and the pulse oximeter. With reference to FIG. 1, a stereo diagram of a conventional blood glucose meter is illustrated. As shown in FIG. 1, a user is able to insert a strip 2' into the conventional blood glucose meter 1', and then, after a blood of the user is dropped to a reacting zone 21' of the strip 2', the blood glucose meter 1' starts to measure, calculate, and show the concentration value of the blood glucose on a screen 11'.

Generally, the blood glucose meter 1' shown in FIG. 1 can only show the concentration value of the blood glucose on the screen 11', so that the user needs to use other electronic devices, for example, a computer, for recording and tracing the daily concentration value of the blood glucose. However, that is inconvenient for the user to use the computer to record and trace the daily concentration value of the blood glucose. On another aspect, with the development of technology, the relevance between 3C products, biotechnologies and medical information is getting increasing, in which, that includes interdisciplinary technologies such as images, database and network. For the above reason, it knows that is important to integrate the information of the biomedical device and the 3C products for facilitating the user.

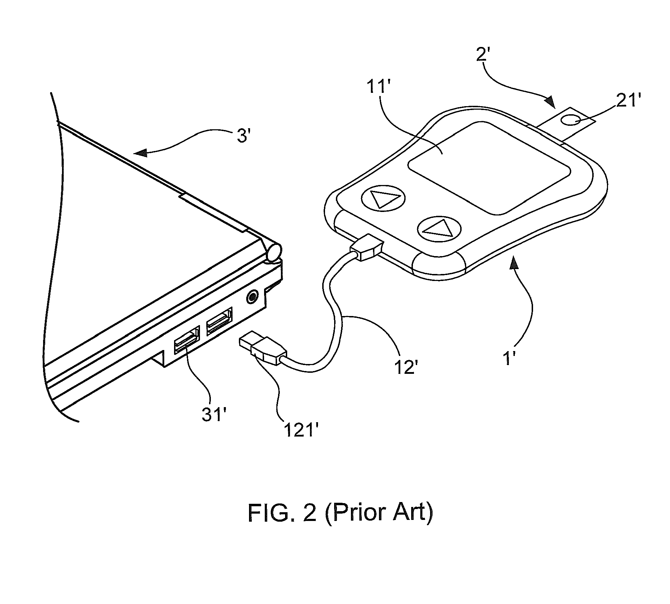

Therefore, the vendors of the biomedical devices provide a new blood glucose meter capable of executing the data transmission with the computer. Referring to FIG. 2, a schematic view of the blood glucose meter communicating with the computer is illustrated. As shown in FIG. 2, a connection cable 12' is added to the blood glucose meter 1', wherein the connection cable 12' has a USB connector able to be inserted into a USB slot 31' of the computer 3', then the user is able to upload the daily concentration value of the blood glucose to the computer 3' through an application software installed in the computer 3', and then the daily concentration value of the blood glucose can be recorded and traced automatically. Therefore, by connecting the blood glucose 1' with the computer 3', it is easily to record and trace the daily blood glucose concentration value for the user.

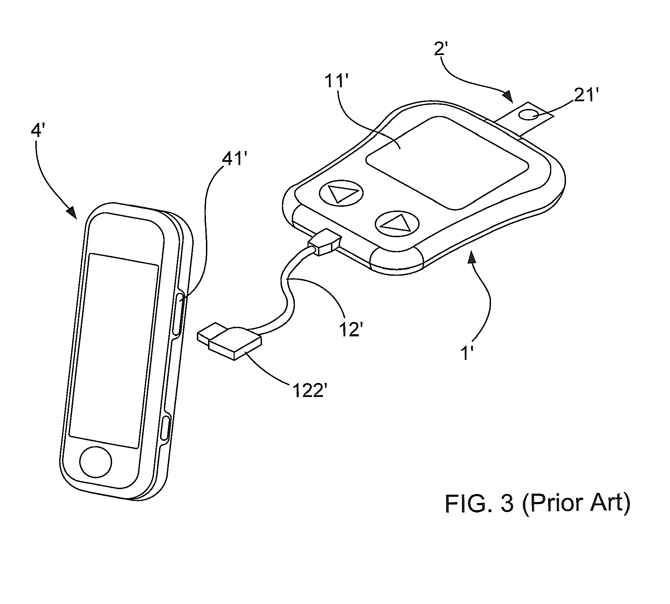

Although the blood glucose meter 1' with the connection cable 12' is able to execute the data transmission and the information integration with the computer 3', it still has a problem for an elderly user. The problem is that the elderly user can not carry the computer 3' everywhere whatever the computer 3' is a desktop PC or a notebook. Thus, to solve the problem, a feasible way is to make the blood glucose meter able to execute the data transmission and the information integration with the popular portable electronic device, for instance, a smart phone. Referring to FIG. 3, the schematic view of the blood glucose meter communicating with the smart phone is illustrated. As shown in FIG. 3, similarly, the blood glucose meter 1' connecting to the smart phone 4' via the connection cable 12'; however, the difference is that the connection cable 12' has a specific connector 122' but not the USB connector 121'. So that, after the blood glucose meter 1' is connected to the smart phone 4' by means of the connection cable 12' and the specific connector 122', the user can unload the daily blood glucose concentration value to the smart phone 4'; Besides, since the smart phone 4' is thin and light, so that does not cause the burden for the elderly user to carry the smart phone 4' everywhere.

However, there are various brands of the smart phones, wherein in two different brands of the smart phones, the format of two distinguished data-transmitting slots is different. Therefore, when the user purchases a new smart phone, and the brand of the new smart phone is different from the old smart phone of the user, such that the blood glucose meter can not communicate with the new smart phone by using the old connection cable. In addition, even if the user buys a new connection cable capable of connecting to the new smart phone, it is not sure that the blood glucose meter can communicate with the new smart phone. Actually, it needs to pass the certification of the data-transmuting format defined by vender of the new smart phone, and then the blood glucose meter is allowed to execute the data transmission and the information integration with the new smart phone.

Thus, according to the above description, it knows that is not easy to make the blood glucose meter (i.e., the biomedical device) execute the data transmission and the information integration with the smart phone (i.e., the portable electronic device). So that, in order to make the data transmission and the information integration being performed between the biomedical device and the portable electronic device more easily, the inventors of the present application have made great efforts to make inventive research thereon and eventually provided a biomedical device capable of using an earphone and microphone plug to transmit data and a method for transmitting data.

### BRIEF SUMMARY OF THE INVENTION

The first objective of the present invention is to provide a biomedical device capable of using an earphone and microphone plug to transmit data, the biomedical device has a standard earphone and microphone plug which can be inserted into an earphone and microphone jack of a potable electronic device, such that, without passing the certification of the transmission format defined by a potable electronic device vender in advance, the biomedical device is able to communicate with the portable electronic device, and the data transmission and the information integration between the biomedical device and the portable electronic device is finished.

The second objective of the present invention is to provide a biomedical device capable of using an earphone and microphone plug to transmit data, the biomedical device has a power management unit capable of obtaining an external power source from a battery or a portable electronic device, so as to transform the external power source to the required system power of the biomedical device, so that, it not needs to worry about the power shortage when the biomedical device is used.

The third objective of the present invention is to provide a method for using an earphone and microphone plug to transmit biomedical data, through the method, a user can not only use a biomedical device to measure the self biomedical information, but also input the biomedical data into a portable electronic device by way of the data transmission and the information integration between the biomedical device and the portable electronic device, so that the portable electronic device can be further used to record and trace the daily biomedical data of the user.

Accordingly, to achieve the first objective and the second objective of the present invention, the inventors propose a biomedical device capable of using an earphone and microphone plug to transmit data, comprising: a measuring unit, a micro-control unit, an earphone and microphone plug, a switch unit, a level shift unit, an amplifying unit, and a power management unit.

The measuring unit can connect to an object under test and measure biomedical information of the object under test. The micro-control unit couples to the measuring unit for receiving the biomedical information, so as to process the biomedical information to biomedical data. The earphone and microphone plug can be inserted into an earphone and microphone jack of a portable electronic device, and has a microphone input terminal, a ground terminal, a left channel output terminal, and a right channel output terminal, wherein the microphone input terminal couples to the micro-control unit.

The switch unit couples to the right channel output terminal and the micro-control unit, wherein when the earphone and microphone plug is inserted into the earphone and microphone jack of the portable electronic device, the switch unit executing the signal switch to stop the portable electronic device outputting audio signal, and then the micro-control unit is able to input the biomedical data into the portable electronic device through the microphone input terminal. The level shift unit couples to the switch unit and the micro-control unit, and receives the audio signal via the switch unit, after receiving the audio signal, the level shift unit amplifies the audio signal, converts the audio signal to DC signal, and outputs the DC signal to the micro-control unit.

The amplifying unit couples to the switch unit for receiving and amplifying the audio signal. The power management unit couples to the left channel output terminal and electrically connects to an external power source via the left channel output terminal, the power management unit is used to transform the external power source to a system power for driving the measuring unit, the micro-control unit, the switch unit, the level shift unit, and the amplifying unit.

Wherein after the earphone and microphone plug is inserted into the earphone and microphone jack, by means of the earphone and microphone plug, the portable electronic device transmits a configuration data to the micro-control unit via the switch unit; after that, the micro-control unit outputs an identification code to the portable electronic device through the microphone input terminal, and then an application software installed in the portable electronic device determines the identification code for checking the communication between the micro-control unit and the portable electronic device.

Moreover, to achieve the third objective of the present invention, the inventors propose a method for using an earphone and microphone plug to transmit biomedical data, the method comprises the steps of:
(1) inserting an earphone and microphone plug of a biomedical device into an earphone and microphone jack of a portable electronic device ;
(2) through a right channel output terminal of the earphone and microphone plug, the portable electronic device outputs a configuration data to a micro-control unit of the biomedical device;
(3) the micro-control unit receiving and analyzes the configuration data;
(4) the micro-control unit outputs a identification code to the portable electronic device via a microphone input terminal of the earphone and microphone plug;
(5) an application software installed in the portable electronic device determines the identification code;
(6) whether the identification code has been determined, if yes, proceeding to step (7), otherwise, proceeding to step (1);
(7) the communication of the micro-control unit and the portable electronic device is established;
(8) determining whether an object under test is connected to a measuring unit of the biomedical device, if yes, proceeding to step (9), otherwise, proceeding to step (8);
(9) a switch unit of the biomedical device stops the portable electronic device outputting audio signal;
(10) determining whether the object under test can be measured, if yes, proceeding to step(11), otherwise, proceeding to step(8);
(11) the measuring unit measures biomedical information of the object under test, and outputs the biomedical information to the micro-control unit;
(12) the biomedical information is processed to biomedical data by the micro-control unit;
(13) the micro-control unit inputs the biomedical data into the portable electronic device via the microphone input terminal;
(14) determining whether the measurement and the record of the biomedical data of the object under test is finished, if yes, proceeding to step(15), otherwise, proceeding to step(8);
(15) removing the object under test from the biomedical device; and
(16) removing the biomedical device from the portable electronic device.

### BRIEF DESCRIPTION OF THE SEVERAL VIEWS OF THE DRAWINGS

The invention as well as a preferred mode of use and advantages thereof will be best understood by referring to the following detailed description of an illustrative embodiment in conjunction with the accompanying drawings, wherein:
FIG. 1 is a stereo diagram of a conventional blood glucose meter;
FIG. 2 is a schematic view of the blood glucose meter communicating with a computer;
FIG. 3 is the schematic view of the blood glucose meter communicating with a smart phone;
FIG. 4 is a framework view of a first embodiment of a biomedical device capable of using an earphone and microphone plug to transmit data according to the present invention;
FIG. 5 is the schematic view of the biomedical device connecting to a smart phone;
FIG. 6 is a front view of an earphone and microphone plug of the biomedical device according to the present invention;
FIG.s 7A, 7B and 7C are signal timing graphic diagrams of the biomedical device executes the data transmission and the information integration with the smart phone;
FIG. 8 is the stereo view of a body fat and blood pressure meter;
FIG. 9 is the front view of an electrocardiography meter;
FIG. 10 is the framework view of a second embodiment of the biomedical device capable of using the earphone and microphone plug to transmit data according to the present invention; and
FIG.s 11A, 11B and 11C are flow charts of a method for using an earphone and microphone plug to transmit biomedical data according to the present invention.

### DETAILED DESCRIPTION OF THE INVENTION

To more clearly describe a biomedical device capable of using an earphone and microphone plug to transmit data and a method for transmitting data according to the present invention, embodiments of the present invention will be described in detail with reference to the attached drawings hereinafter.

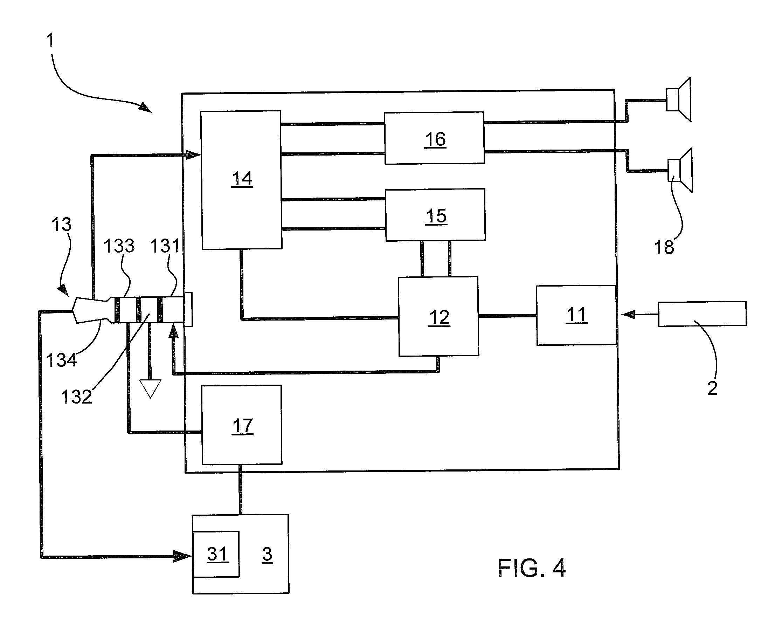

The biomedical device capable of using the earphone and microphone plug to transmit data of the present invention includes two embodiments. With reference to FIG. 4, which illustrates a framework view of a first embodiment of the biomedical device capable of using an earphone and microphone plug to transmit data according to the present invention. As shown in FIG. 4, the first embodiment of the biomedical device 1 includes: a measuring unit 11, a micro-control unit 12, an earphone and microphone plug 13, a switch unit 14, a level shift unit 15, an amplifying unit 16, a power management unit 17, and a speaker unit 18.

The measuring unit 11 is capable of connecting to an object under test 2 and measuring biomedical information of the object under test 2. The micro-control unit 12 couples to the measuring unit 11 for receiving the biomedical information, so as to process the biomedical information to biomedical data. The earphone and microphone plug 13 is able to be inserted into an earphone and microphone jack 31 of a portable electronic device 3, wherein earphone and microphone plug 13 has a microphone input terminal 131, a ground terminal 132, a left channel output terminal 133, and a right channel output terminal 134, and the microphone input terminal 131 couples to the micro-control unit 12.

As shown in FIG.4, The switch unit 14 couples to the right channel output terminal 134 and the micro-control unit 12, wherein when the earphone and microphone plug 13 is inserted into the earphone and microphone jack 31 of the portable electronic device 3, the switch unit 14 executes the signal switch to stop the portable electronic device 3 outputting audio signal, and then the micro-control unit 12 is able to input the biomedical data into the portable electronic device 3 through the microphone input terminal 131. The level shift unit 15 couples to the switch unit 14 and the micro-control unit 12, and is employed to receive the audio signal via the switch unit 14. After receiving the audio signal, the level shift unit 15 amplifies the audio signal, converts the audio signal to DC signal, and outputs the DC signal to the micro-control unit 12.

Referring to FIG. 4 again, the amplifying unit 16 couples to the switch unit 14 for receiving and amplifying the audio signal. The power management unit 17 couples to the left channel output terminal 133, in addition, through the left channel output terminal 133, the power management unit 17 can electrically connect to an external power source. The power management unit 17 is used to transform the external power source to a system power for driving the measuring unit 11, the micro-control unit 12, the switch unit 14, the level shift unit 15, and the amplifying unit 16. In the first embodiment of the biomedical device 1, a battery of the portable electronic device 3 is used as the external power source, so that, after the earphone and microphone plug 13 is inserted into the earphone and microphone jack 31, the power management unit 17 can obtain the power source from the battery of the portable electronic device 3, and then the power management unit 17 transform the power source to the system power. Moreover, in the first embodiment of the biomedical device 1, the speaker unit 18 couples to the amplifying unit 16 for receiving the audio signal amplified by the amplifying unit 16, so as to output the audio signal.

Must be especially noted that, in the framework of the first embodiment of the biomedical device 1 of the present invention, after the earphone and microphone plug 13 is inserted into the earphone and microphone jack 31, through the earphone and microphone plug 13 and the switch unit 14, the portable electronic device 3 is capable of transmitting a configuration data to the micro-control unit 12; after that, the micro-control unit 12 outputs a identification code to the portable electronic device 3, and then an application software installed in the portable electronic device 3 determines the identification code for checking the communication between the micro-control unit 12 and the portable electronic device 3. When the application software successfully detects the identification code, meanwhile, the communication between the micro-control unit 12 and the portable electronic device 3 has been established; therefore, the biomedical device 1 is able to execute the data transmission and the information integration with the portable electronic device 3, so that, through the portable electronic device 3, a user can upload the daily biomedical data thereof measured by the biomedical device 1 to a Cloud Database for a telemedicine management. Furthermore, it must stress that the user is able to download the application software from a network software store provided by vendors of the portable electronic devices, and to install the application software in the portable electronic device.

The characteristic of the biomedical device 1 of the present invention is using the earphone and microphone plus 13 to communicate with the portable electronic device 3; thus, in order to completely disclose the characteristic of the present invention, how the biomedical device 1 execute the data transmission and the information integration with the portable electronic device 3 will be described in follows.

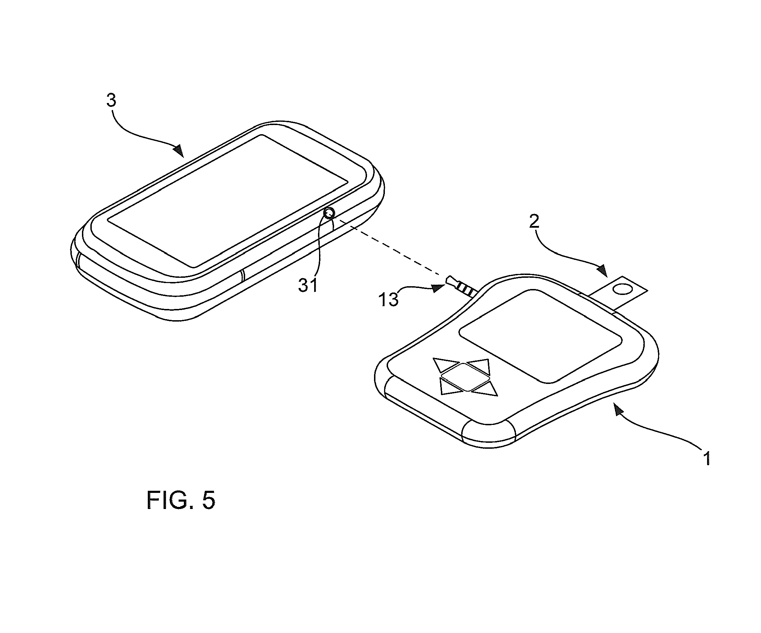

Referring to FIG. 4 again, and simultaneously referring to FIG. 5, which illustrates a schematic view of the biomedical device connecting to a smart phone. As shown in FIG. 4 and FIG. 5, since the biomedical device 1 of the present invention includes the earphone and microphone plug 13, so the user can insert the earphone and microphone plug 13 into the earphone and microphone jack 31 of the portable electronic device 3, so as to establish the communication between the biomedical device 1 and the portable electronic device 3. As shown in FIG. 5, the biomedical device 1 is a blood glucose meter, the portable device 3 is a smart phone, and the object under test 2 is a blood glucose strip, wherein the blood glucose strip has an enzyme 21 used for reacting with a blood of the user. The blood glucose strip, i.e., the object under test 2, can be inserted into a measuring device of the blood glucose meter, i.e., the measuring unit 11 of the biomedical device 1, so that the blood glucose meter is able to measure and calculate the value of the blood glucose concentration. Moreover, the user is able to purchase and download the application software from an android market provided by GOOGLE®, an App Store provided by Apple@, an Ovi Store provided by Nokia® or an App Store provided by BlackBerry®, and to directly install the application software in the smart phone.

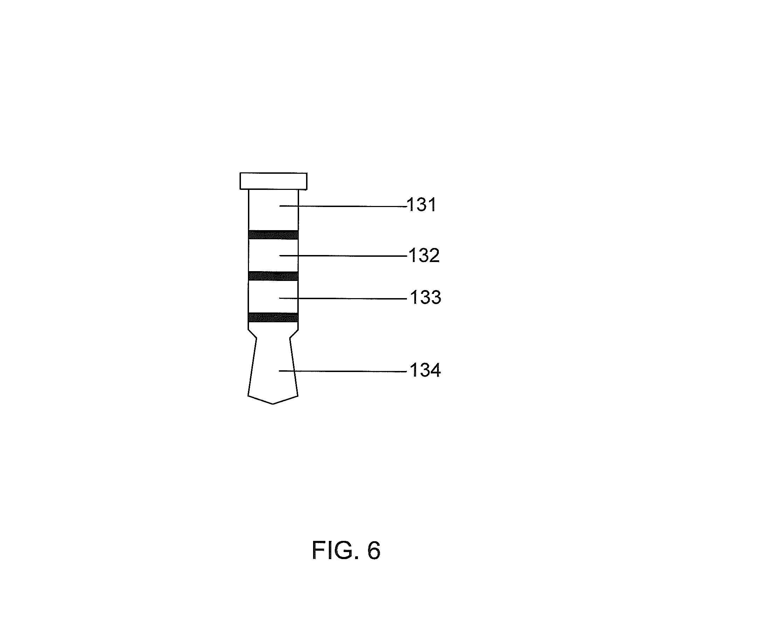

Referring to FIG. 4 , and FIG. 5 again, and simultaneously referring to FIG. 6, which illustrates a front view of the earphone and microphone plug of the biomedical device according to the present invention. As above mentioned, the earphone and microphone plug 13 of the biomedical device 1 has the microphone input terminal 131, the ground terminal 132, the left channel output terminal 133, and the right channel output terminal 134, wherein the microphone input terminal 131 couples to the micro-control unit 12, the right channel output terminal 134 couples to the switch unit 14, and the left channel output terminal 133 couples to the power management unit 17.

In the framework of the first embodiment of the biomedical device 1 of the present invention, the right channel output terminal 134 is used for inputting the audio signal outputted by the smart phone into the switch unit 14, then the switch unit 14 transmits the audio signal to the micro-control unit 12, and then the micro-control unit 12 analyzes the audio signal and calculates a data frequency of the biomedical data. Furthermore, when the biomedical device 1 is connected to the smart phone, the smart phone outputs the configuration data thereof to the micro-control unit 12 via the right channel output terminal 134, then the micro-control unit 12 outputs the identification code to the smart phone through the microphone input terminal 131, and then the application installed in the smart phone determines the identification code for checking the communication between the biomedical device 1 and the smart phone. When the application software successfully detects the identification code, the communication between the biomedical device 1 and the smart phone has been established, and the biomedical device 1 is able to execute the data transmission and the information integration with the smart phone.

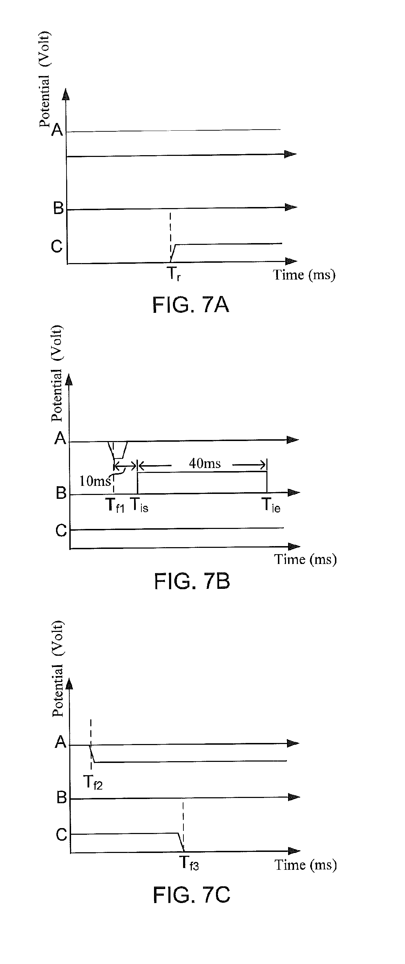

Referring to FIG. 4 and FIG.5 again, and simultaneously referring to FIGs. 7A, 7B and 7C, which illustrate signal timing graphic diagrams of the biomedical device executes the data transmission and the information integration with the smart phone. After the blood glucose meter, i.e., the biomedical device 1 of the present invention, is connected to the smart phone, i.e., the portable electronic device 3, the signal timing graphic diagrams of the blood glucose meter and the smart phone as shown in FIG. 7A, in which signal A is the signal outputted from the left channel output terminal 133, signal B is the signal outputted from the microphone input terminal 131, and signal C is the signal inputted into the micro-control unit 12 by the switch unit 14. In addition, as shown in FIG 7A, before the blood glucose, i.e., the object under test, is inserted into the blood glucose meter, the signal A is kept at a high potential level, the signal B and the signal C are maintained at a low potential level; however, after the blood glucose strip is inserted into the blood glucose meter, the signal A is still kept at the high potential level, the signal B is still maintained at the low potential level, but the signal C rises to the high potential level at the time coordinate of Tr.

Furthermore, after the blood of the user is dropped to the enzyme 21, the blood glucose meter measure and calculate the value of the blood glucose concentration, in which the value of the blood glucose concentration is the biomedical data; moreover, since the sampling frequency of the audio signal outputted by the smart phone is 44.1 kHz, so that, through the microphone input terminal 131, the micro-control unit 12 outputs the biomedical data to the smart phone by the data frequency of 10 kHz, and the data format of the biomedical data is binary code.

As shown in FIG. 7B, when the biomedical data is inputted into the smart phone, the signal A firstly falls at time coordinate of Tf1, and after a period of time, the signal A rises. When the signal A falls and after 10 ms, the biomedical data, i.e., the calculated value of the blood glucose concentration, begins to be inputted into the smart phone at time coordinate of Tis, and then the input of the biomedical data is finished at time coordinate of Tie. As shown in FIG. 7B, the time differential of Tie and Tis is 40 ms. After the input of the biomedical data is finished, as shown in FIG. 7C, the signal A falls to the low potential level at time coordinate of Tf2; furthermore, after the blood glucose strip 21 is removed from the blood glucose meter, the signal C falls to the low potential level at time coordinate of Tf3.

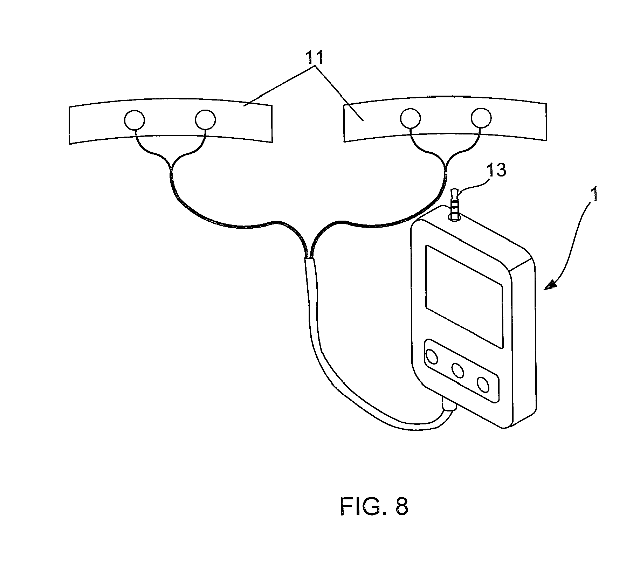

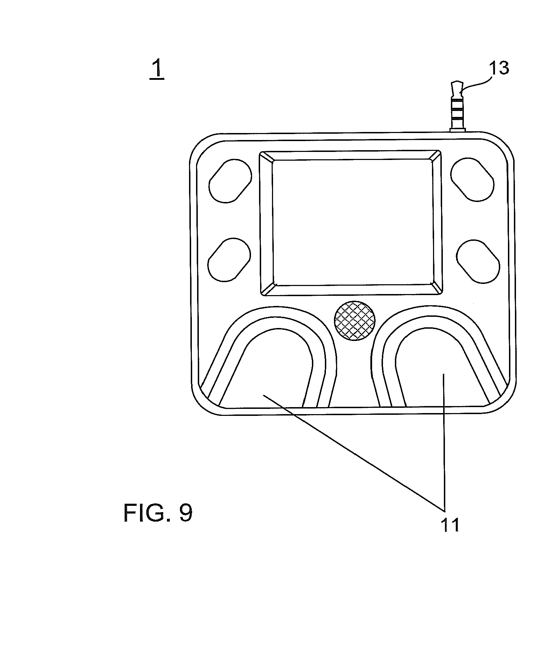

In the above mention, that uses the blood glucose meter as the first embodiment of the biomedical device 1 to describe how the biomedical device of the present invention execute the data transmission and the information integration with the smart phone. However, since the present body fat meter, the blood pressure meter, the pulse oximeter, and the electrocardiography meter have been miniaturized, so that the biomedical device of the present invention can not only applied to the blood meter, but also be implemented to a body fat and blood pressure meter, a pulse oximeter and an electrocardiography meter. Please refer to FIG. 8, which illustrates a stereo view of the body fat and blood pressure meter, as shown in FIG. 8, the measuring unit 11 is two ring electrodes which are able to measure the body fat and the blood pressure of the user by way of surrounding the left wrist and the right wrist of the user, respectively. Moreover, please refer to FIG. 9, which illustrates the front view of an electrocardiography meter, as shown in FIG. 9, the biomedical device 1 of the present invention also can be the electrocardiography meter, in which the measuring unit 11 is two electrodes. The user is able to place two fingers thereof on the two electrodes, respectively, such that the electrocardiography meter can measure and plot an electrocardiography of the user.

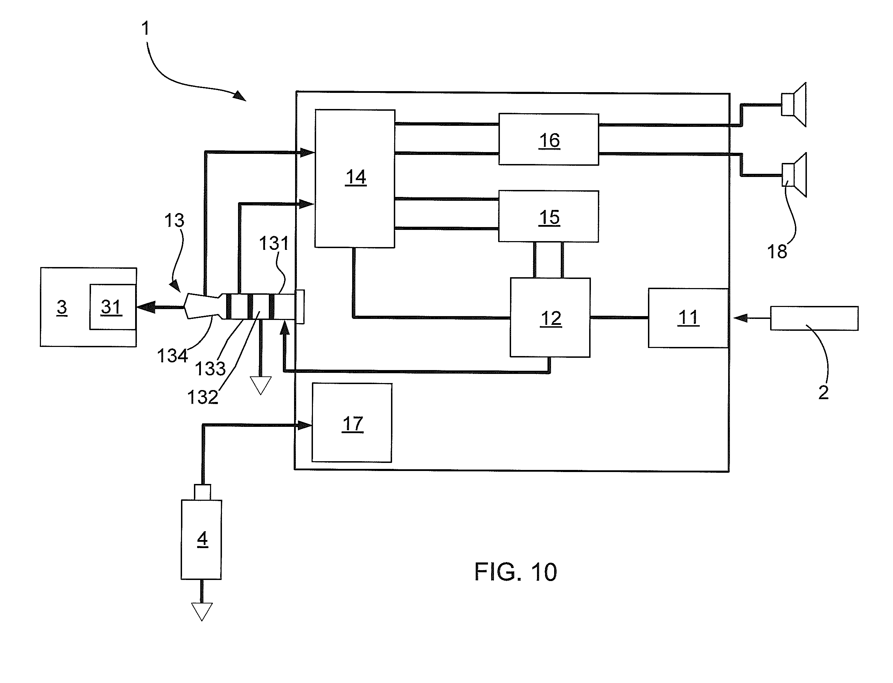

Moreover, the biomedical device capable of using the earphone and microphone plug to transmit data of the present invention further includes a second embodiment. Please refer to FIG. 10, which illustrates framework view of the second embodiment of the biomedical device according to the present invention. As shown in FIG. 10, the framework of the second embodiment of the biomedical device includes: the measuring unit 11, the micro-control unit 12, the earphone and microphone plug 13, the switch unit 14, the level shift unit 15, the amplifying unit 16, the power management unit 17, and the speaker unit 16.

Referring to FIG. 10 again, and simultaneously referring to FIG. 4, compared with the first embodiment, in the framework of the second embodiment of the biomedical device 1, the switch unit 14 couples to the left channel output terminal 133, the right channel output terminal 134 and the micro-control unit 12; in addition, the power management unit 17 only electrically connects to the external power source and not couples to the left channel output terminal 133. In the second embodiment, since the left channel output terminal 133 not needs to couple to the power management unit 17 for supplying the external power source, both the left channel output terminal 133 and the right channel output terminal 134 are used for outputting the audio signal to the biomedical device 1, or employed to transmit the configuration data of the portable electronic device 3 to the micro-control unit 12. Moreover, the same to the first embodiment, not only being applied to the blood meter, the second embodiment of the biomedical device 1 can also be implemented to the body fat and blood pressure meter, the pulse oximeter and the electrocardiography meter.

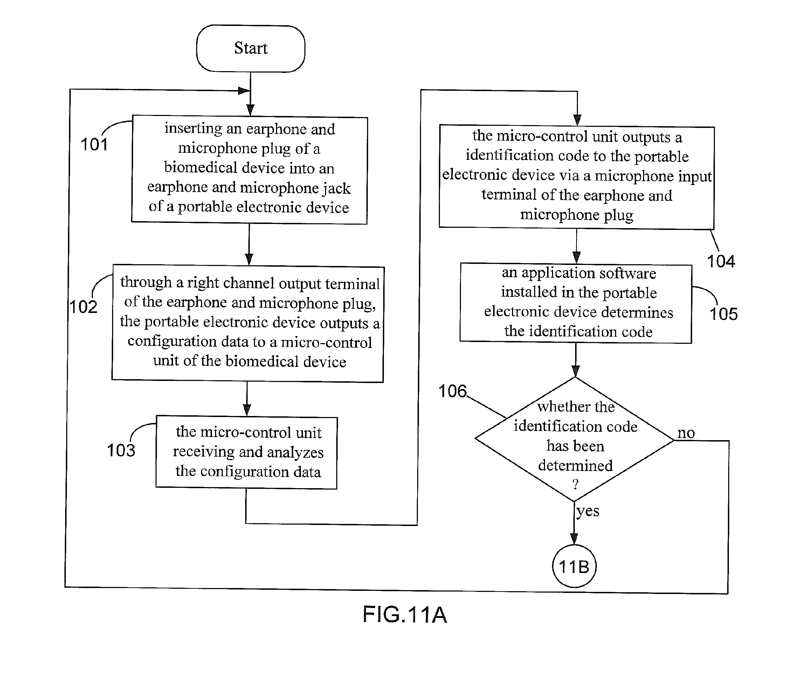

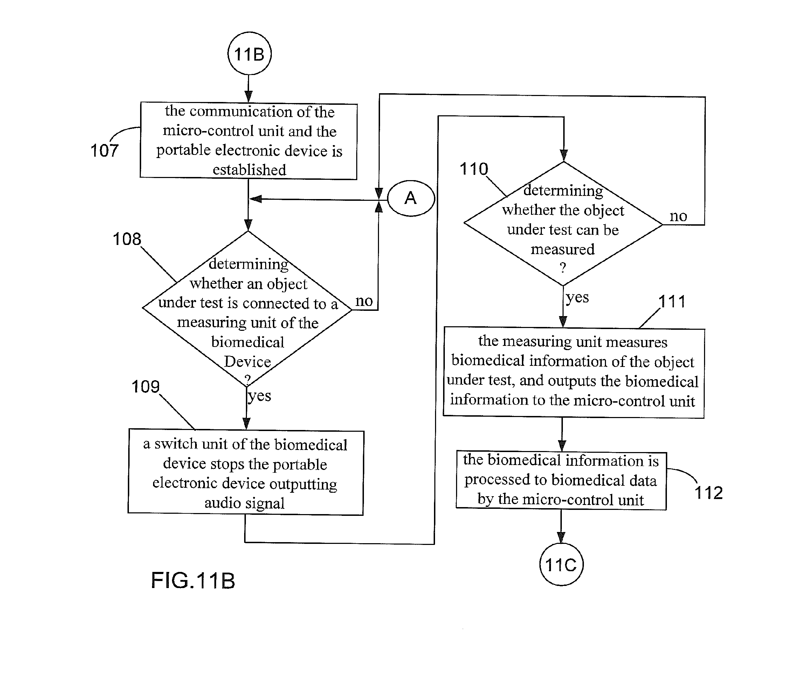

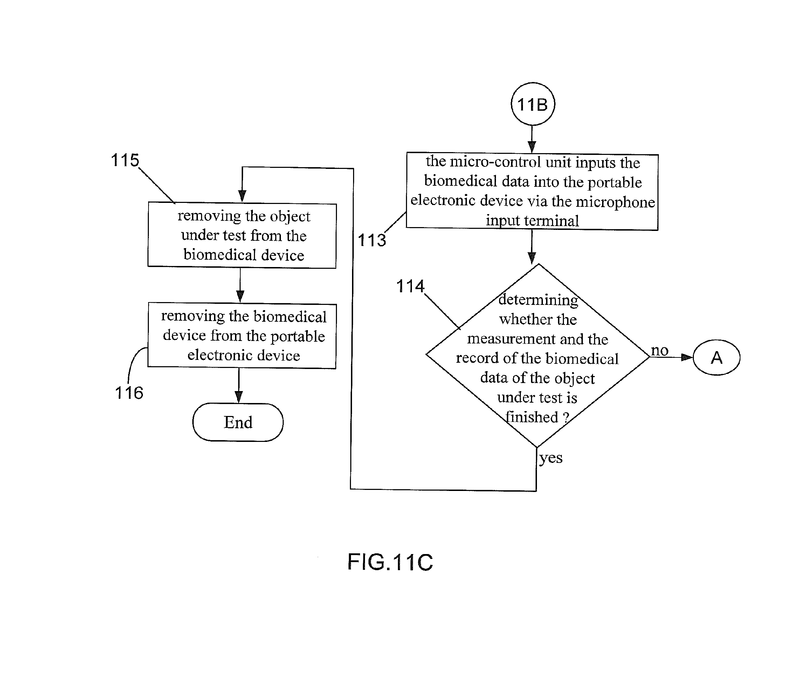

Through the above description, the biomedical device capable of using the earphone and microphone plug to transmit data according to the present invention has been clearly disclosed; in addition, through the flow charts, the method for using the earphone and microphone plug to transmit the biomedical data will be disclosed in follows. With reference to FIGs. 11A, 11B and 11C, which illustrate the flow charts of the method for using the earphone and microphone plug to transmit the biomedical data according to the present invention. As shown in FIG. 11A, FIG. 11B and FIG. 11C, the method for using the earphone and microphone plug to transmit the biomedical data includes the steps of:

Firstly, proceeding step (101), inserting the earphone and microphone plug 13 of the biomedical device 1 into the earphone and microphone jack 31 of the portable electronic device 3; Next proceeding step (102) through the right channel output terminal 134 of the earphone and microphone plug 13, the portable electronic device 3 outputs the configuration data to the micro-control unit 12 of the biomedical device 1; Then proceeding to step (103), the micro-control unit 12 receiving and analyzing the configuration data. After the micro-control unit 12 finishes the analysis of the configuration data, then proceeding to step (104), the micro-control unit 12 outputs the identification code to the portable electronic device 3 via the microphone input terminal 131 of the earphone and microphone plug 13; Next proceeding to step (105), the application software installed in the portable electronic device 3 determining the identification code; Next proceeding to step (106), whether the identification code has been determined, if yes, proceeding to step (7), the communication of the micro-control unit 12 and the portable electronic device 3 is established; if no, proceeding back to step (101).

After the communication of the micro-control unit 12 and the portable electronic device 3 has been established, the flow of the method proceeds to step (108), determining whether the object under test 2 is connected to the measuring unit 11 of the biomedical device 1, if yes, proceeding to step (109), the switch unit 14 of the biomedical device 1 stops the portable electronic device 3 outputting the audio signal; if no, that means there is no object under test 2 being connected to the measuring unit 11 of the biomedical device 1, such that proceeding to step (108).

Furthermore, after the object under test 2 is connected to the measuring unit 11, then proceeding to step (110), determining whether the object under test 2 can be measured, if yes, proceeding to step (111), the measuring unit 11 measures the biomedical information of the object under test 2, and outputs the biomedical information to the micro-control unit 12; if no, proceeding to step (108). Next proceeding to step (112), the biomedical information is processed to biomedical data by the micro-control unit 12, and then proceeding to step (113), the micro-control unit 12 inputs the biomedical data into the portable electronic device 3 via the microphone input terminal 131.

After steps (101) to (113) are finished, the biomedical device 1 executes the data transmission and the information integration with the portable electronic device 3; therefore, proceeding to step (114), determining whether the measurement and the record of the biomedical data of the object under test 2 is finished, if yes, proceeding to step (115), removing the object under test 2 from the biomedical device 1; if no, proceeding to step (108). Finally, after step (115) is completed, next proceeding to step (116), removing the biomedical device 1 from the portable electronic device 3.

Thus, though the steps (101) to (116), the user can not only utilize the biomedical device 1 to measure the self biomedical information, but also input the biomedical data into the portable electronic device 3 by way of the data transmission and the information integration between the biomedical device 1 and the portable electronic device 3, so that the portable electronic device 3 can be further used to record and trace the daily biomedical data of the user.

So, through the first embodiment, the second embodiment and the flow charts, the biomedical device capable of using the earphone and microphone plug to transmit data and the method for transmitting data according to the present invention has been disclosed completely and clearly in the above description. In summary, the present invention has the following advantages:

1. The biomedical device of the present invention has one earphone and microphone plug capable of being inserted into one earphone and microphone jack of the portable electronic device, such that, by means of the earphone and microphone plug and the application software installed in the portable electronic device, the biomedical device of the present invention is able to execute the data transmission and the information integration with the portable electronic device.

2. Inheriting above point 1, by way of the earphone and microphone plug being inserted into the earphone and microphone jack of the potable electronic device, such that, without passing the certification of the transmission format defined by the potable electronic device vender in advance, the biomedical device is able to communicate with the portable electronic device, and the data transmission and the information integration between the biomedical device and the portable electronic device is achieved; it is very convenient for the user.

3. Inheriting above point 1, the user is able to purchase and download the application software from an android market provided by GOOGLE®, an App Store provided by Apple®, an Ovi Store provided by Nokia® or an App Store provided by BlackBerry®, and to directly install the application software in the portable electronic device (the smart phone).

4. The system power of the biomedical device of the present invention is provide by the battery, the power supply or the portable electronic device, so that the user not needs to worry about the power shortage when using the biomedical device.

5. Through the method for using the earphone and microphone plug to transmit the biomedical data, the user can not only use the biomedical device to measure the self biomedical information, but also input the biomedical data into the portable electronic device by way of the data transmission and the information integration between the biomedical device and the portable electronic device, so that the portable electronic device can be further used to record and trace the daily biomedical data of the user.

The above description is made on embodiments of the present invention. However, the embodiments are not intended to limit scope of the present invention, and all equivalent implementations or alterations within the spirit of the present invention still fall within the scope of the present invention.

### CLAUSES

1. A biomedical device capable of using an earphone and microphone plug to transmit data, comprising:
   a measuring unit, capable of connecting to an object under test and measuring biomedical information of the object under test;
   a micro-control unit, coupling to the measuring unit for receiving the biomedical information, so as to process the biomedical information to biomedical data;
   an earphone and microphone plug, capable of being inserted into an earphone and microphone jack of a portable electronic device, and having a microphone input terminal, a ground terminal, a left channel output terminal, and a right channel output terminal, wherein the microphone input terminal couples to the micro-control unit;
   a switch unit, coupling to the right channel output terminal and the micro-control unit, wherein when the earphone and microphone plug is inserted into the earphone and microphone jack of the portable electronic device, the switch unit executing the signal switch to stop the portable electronic device outputting audio signal, and then the micro-control unit
   is able to input the biomedical data into the portable electronic device through the microphone input terminal;
   a level shift unit, coupling to the switch unit and the micro-control unit, and receiving the audio signal via the switch unit, after receiving the audio signal, the level shift unit amplifying the audio signal, converting the audio signal to DC signal, and outputting the DC signal to the micro-control unit;
   an amplifying unit, coupling to the switch unit for receiving and amplifying the audio signal; and
   a power management unit, coupling to the left channel output terminal and electrically connecting to an external power source through the left channel output terminal, the power management unit being used to transform the external power source to a system power for driving the measuring unit, the micro-control unit, the switch unit, the level shift unit, and the amplifying unit;
   wherein after the earphone and microphone plug is inserted into the earphone and microphone jack, by means of the earphone and microphone plug, the portable electronic device transmitting a configuration data to the micro-control unit via the switch unit, after that, the micro-control unit outputting an identification code to the portable electronic device through the microphone input terminal, and then an application software installed in the portable electronic device determines the identification code for checking the communication between the micro-control unit and the portable electronic device.
2. The biomedical device capable of using the earphone and microphone plug to transmit data of Clause 1, further comprising a speaker unit, coupling to the amplifying unit for receiving the audio signal which has been amplified and outputting the audio signal.
3. The biomedical device capable of using the earphone and microphone plug to transmit data of Clause 1, wherein when the earphone and microphone plug is inserted into the earphone and microphone jack of the portable electronic device, meanwhile, the signal outputted from the left channel output terminal being maintained at a high level, so that the power management unit obtains the external power source.
4. The biomedical device capable of using the earphone and microphone plug to transmit data of Clause 1, wherein the measuring unit is selected from the group consisting of: a measuring device of blood glucose strip, a measuring device of body fat, a measuring device of blood pressure, and a measuring device of electrocardiography.
5. The biomedical device capable of using the earphone and microphone plug to transmit data of Clause 4, wherein the object under test is selected from the group consisting of: a blood glucose strip and a man body.
6. The biomedical device capable of using the earphone and microphone plug to transmit data of Clause 1, wherein the micro-control unit inputs the biomedical data into the portable electronic device by a data frequency of 10 kHz through the microphone input terminal.
7. The biomedical device capable of using the earphone and microphone plug to transmit data of Clause 6, wherein the data format of biomedical data inputted into the portable electronic device is binary code.
8. The biomedical device capable of using the earphone and microphone plug to transmit data of Clause 1, wherein the portable electronic device is selected from the group consisting of: a smart phone, a PDA, a portable game console, a digital photo frame, a notebook, a netbook, a tablet PC, and an iPAD.
9. A biomedical device capable of using an earphone and microphone plug to transmit data, comprising:
   a measuring unit, capable of connecting to an object under test and measuring biomedical information of the object under test;
   a micro-control unit, coupling to the measuring unit for receiving the biomedical information,
   so as to process the biomedical information to biomedical data;
   an earphone and microphone plug, capable of being inserted into an earphone and microphone jack of a portable electronic device, and having a microphone input terminal, a ground terminal, a left channel output terminal, and a right channel output terminal, wherein the microphone input terminal couples to the micro-control unit;
   a switch unit, coupling to the left channel output terminal, the right channel output terminal and the micro-control unit, wherein when the earphone and microphone plug is inserted into the earphone and microphone jack of the portable electronic device, the switch unit executing the signal switch to stop the portable electronic device outputting audio signal, and then the micro-control unit is able to input the biomedical data into the portable electronic device through the microphone input terminal;
   a level shift unit, coupling to the switch unit and the micro-control unit, and receiving the audio signal via the switch unit, after receiving the audio signal, the level shift unit amplifying the audio signal, converting the audio signal to DC signal, and outputting the DC signal to the micro-control unit;
   an amplifying unit, coupling to the switch unit for receiving and amplifying the audio signal; and
   a power management unit, electrically connecting to an external power source and being employed to transform the external power source to a system power, such that the measuring unit, the micro-control unit, the switch unit, the level shift unit, and the amplifying unit can be driven by the system power;
   wherein after the earphone and microphone plug is inserted into the earphone and microphone jack, by way of the earphone and microphone plug, the portable electronic device transmitting a configuration data to the micro-control unit via the switch unit, after that, the micro-control unit outputting an identification code to the portable electronic device through the microphone input terminal, and then an application software installed in the portable electronic device determines the identification code for checking the communication between the micro-control unit and the portable electronic device.
10. The biomedical device capable of using the earphone and microphone plug to transmit data of Clause 9, further comprising a speaker unit, coupling to the amplifying unit for receiving the audio signal which has been amplified and outputting the audio signal.
11. The biomedical device capable of using the earphone and microphone plug to transmit data of Clause 9, wherein when external power source is selected from the group consisting of: a battery and a power supply.
12. The biomedical device capable of using the earphone and microphone plug to transmit data of Clause 1, wherein the measuring unit is selected from the group consisting of: a measuring device of blood glucose strip, a measuring device of body fat, a measuring device of blood pressure, and a measuring device of electrocardiography.
13. The biomedical device capable of using the earphone and microphone plug to transmit data of Clause 12, wherein the object under test is selected from the group consisting of: a blood glucose strip and a man body.
14. The biomedical device capable of using the earphone and microphone plug to transmit data of Clause 9, wherein the micro-control unit inputs the biomedical data into the portable electronic device by a data frequency of 10 kHz through the microphone input terminal; moreover, the format of the biomedical data is binary code.
15. The biomedical device capable of using the earphone and microphone plug to transmit data of Clause 9, wherein the portable electronic device is selected from the group consisting of: a smart phone, a PDA, a portable game console, a digital photo frame, a notebook, a netbook, a tablet PC, and an iPAD.
16. A method for using an earphone and microphone plug to transmit biomedical data, the method comprising the steps of:
   (1) inserting an earphone and microphone plug of a biomedical device into an earphone and microphone jack of a portable electronic device ;
   (2) through a right channel output terminal of the earphone and microphone plug, the portable electronic device outputting a configuration data to a micro-control unit of the biomedical device;
   (3) the micro-control unit receiving and analyzing the configuration data;
   (4) the micro-control unit outputting a identification code to the portable electronic device via a microphone input terminal of the earphone and microphone plug;
   (5) an application software installed in the portable electronic device determining the identification code;
   (6) whether the identification code has been determined, if yes, proceeding to step (7), otherwise, proceeding to step (1);
   (7) the communication of the micro-control unit and the portable electronic device being established;
   (8) determining whether an object under test is connected to a measuring unit of the biomedical device, if yes, proceeding to step (9), otherwise, proceeding to step (8);
   (9) a switch unit of the biomedical device stopping the portable electronic device outputting audio signal;
   (10) determining whether the object under test can be measured, if yes, proceeding to step(11), otherwise, proceeding to step(8);
   (11) the measuring unit measuring biomedical information of the object under test, and outputting the biomedical information to the micro-control unit;
   (12) the biomedical information being processed to biomedical data by the micro-control unit;
   (13) the micro-control unit inputting the biomedical data into the portable electronic device via the microphone input terminal;
   (14) determining whether the measurement and the record of the biomedical data of the object under test is finished, if yes, proceeding to step(15), otherwise, proceeding to step(8);
   (15) removing the object under test from the biomedical device; and
   (16) removing the biomedical device from the portable electronic device.
17. The method for using the earphone and microphone plug to transmit the biomedical data of Clause 16, wherein the measuring unit is selected from the group consisting of: a measuring device of blood glucose strip, a measuring device of body fat, a measuring device of blood pressure, and a measuring device of electrocardiography.
18. The method for using the earphone and microphone plug to transmit the biomedical data of Clause 16, wherein the object under test is selected from the group consisting of: a blood glucose strip and a man body.
19. The method for using the earphone and microphone plug to transmit the biomedical data of Clause 16, wherein the micro-control unit inputs the biomedical data into the portable electronic device by a data frequency of 10 kHz through the microphone input terminal; moreover, the format of the biomedical data is binary code.
20. The method for using the earphone and microphone plug to transmit the biomedical data of Clause 16, wherein the portable electronic device is selected from the group consisting of:
   a smart phone, a PDA, a portable game console, a digital photo frame, a notebook, a netbook, a tablet PC, and an iPAD.

## Claims

1. A biomedical device capable of using an earphone and microphone plug to transmit data, comprising:
a measuring unit, capable of connecting to an object under test and measuring biomedical information of the object under test;
a micro-control unit, coupling to the measuring unit for receiving the biomedical information, so as to process the biomedical information to biomedical data;
an earphone and microphone plug, capable of being inserted into an earphone and microphone jack of a portable electronic device, and having a microphone input terminal, a ground terminal, a left channel output terminal, and a right channel output terminal, wherein the microphone input terminal couples to the micro-control unit;
a switch unit, coupling to the right channel output terminal and the micro-control unit, wherein when the earphone and microphone plug is inserted into the earphone and microphone jack of the portable electronic device, the switch unit executing the signal switch to stop the portable electronic device outputting audio signal, and then the micro-control unit is able to input the biomedical data into the portable electronic device through the microphone input terminal;
a level shift unit, coupling to the switch unit and the micro-control unit, and receiving the audio signal via the switch unit, after receiving the audio signal, the level shift unit amplifying the audio signal, converting the audio signal to DC signal, and outputting the DC signal to the micro-control unit;
an amplifying unit, coupling to the switch unit for receiving and amplifying the audio signal; and
a power management unit, coupling to the left channel output terminal and electrically connecting to an external power source through the left channel output terminal, the power management unit being used to transform the external power source to a system power for driving the measuring unit, the
micro-control unit, the switch unit, the level shift unit, and the amplifying unit;
wherein after the earphone and microphone plug is inserted into the earphone and microphone jack, by means of the earphone and microphone plug, the portable electronic device transmitting a configuration data to the micro-control unit via the switch unit, after that, the micro-control unit outputting an identification code to the portable electronic device through the microphone input terminal, and then an application software installed in the portable electronic device determines the identification code for checking the communication between the micro-control unit and the portable electronic device.

2. The biomedical device capable of using the earphone and microphone plug to transmit data of Claim 1, further comprising a speaker unit, coupling to the amplifying unit for receiving the audio signal which has been amplified and outputting the audio signal.

3. The biomedical device capable of using the earphone and microphone plug to transmit data of Claim 1, wherein when the earphone and microphone plug is inserted into the earphone and microphone jack of the portable electronic device, meanwhile, the signal outputted from the left channel output terminal being maintained at a high level, so that the power management unit obtains the external power source.

4. The biomedical device capable of using the earphone and microphone plug to transmit data of Claim 1, wherein the measuring unit is selected from the group consisting of a measuring device of blood glucose strip, a measuring device of body fat, a measuring device of blood pressure, and a measuring device of electrocardiography.

5. The biomedical device capable of using the earphone and microphone plug to transmit data of Claim 4, wherein the object under test is selected from the group consisting of: a blood glucose strip and a man body.

6. The biomedical device capable of using the earphone and microphone plug to transmit data of Claim 1, wherein the micro-control unit inputs the biomedical data into the portable electronic device by a data frequency of 10 kHz through the microphone input terminal.

7. The biomedical device capable of using the earphone and microphone plug to transmit data of Claim 6, wherein the data format of biomedical data inputted into the portable electronic device is binary code.

8. The biomedical device capable of using the earphone and microphone plug to transmit data of Claim 1, wherein the portable electronic device is selected from the group consisting of: a smart phone, a PDA, a portable game console, a digital photo frame, a notebook, a netbook, a tablet PC, and an iPAD.

9. A biomedical device capable of using an earphone and microphone plug to transmit data, comprising:
a measuring unit, capable of connecting to an object under test and measuring biomedical information of the object under test;
a micro-control unit, coupling to the measuring unit for receiving the biomedical information, so as to process the biomedical information to biomedical data;
an earphone and microphone plug, capable of being inserted into an earphone and microphone jack of a portable electronic device, and having a microphone input terminal, a ground terminal, a left channel output terminal, and a right channel output terminal, wherein the microphone input terminal couples to the micro-control unit;
a switch unit, coupling to the left channel output terminal, the right channel output terminal and the micro-control unit, wherein when the earphone and microphone plug is inserted into the earphone and microphone jack of the portable electronic device, the switch unit executing the signal switch to stop the portable electronic device outputting audio signal, and then the micro-control unit is able to input the biomedical data into the portable electronic device through the microphone input terminal;
a level shift unit, coupling to the switch unit and the micro-control unit, and receiving the audio signal via the switch unit, after receiving the audio signal, the level shift unit amplifying the audio signal, converting the audio signal to DC signal, and outputting the DC signal to the micro-control unit;
an amplifying unit, coupling to the switch unit for receiving and amplifying the audio signal; and
a power management unit, electrically connecting to an external power source and being employed to transform the external power source to a system power, such that the measuring unit, the micro-control unit, the switch unit, the level shift unit, and the amplifying unit can be driven by the system power;
wherein after the earphone and microphone plug is inserted into the earphone and
microphone jack, by way of the earphone and microphone plug, the portable electronic device transmitting a configuration data to the micro-control unit via the switch unit, after that, the micro-control unit outputting an identification code to the portable electronic device through the microphone input terminal, and then an application software installed in the portable electronic device determines the identification code for checking the communication between the micro-control unit and the portable electronic device.

10. The biomedical device capable of using the earphone and microphone plug to transmit data of Claim 9, further comprising a speaker unit, coupling to the amplifying unit for receiving the audio signal which has been amplified and outputting the audio signal.

11. The biomedical device capable of using the earphone and microphone plug to transmit data of Claim 9, wherein when external power source is selected from the group consisting of: a battery and a power supply.

12. The biomedical device capable of using the earphone and microphone plug to transmit data of Claim 1, wherein the measuring unit is selected from the group consisting of: a measuring device of blood glucose strip, a measuring device of body fat, a measuring device of blood pressure, and a measuring device of electrocardiography.

13. The biomedical device capable of using the earphone and microphone plug to transmit data of Claim 12, wherein the object under test is selected from the group consisting of: a blood glucose strip and a man body.

14. The biomedical device capable of using the earphone and microphone plug to transmit data of Claim 9, wherein the micro-control unit inputs the biomedical data into the portable electronic device by a data frequency of 10 kHz through the microphone input terminal; moreover, the format of the biomedical data is binary code.

15. The biomedical device capable of using the earphone and microphone plug to transmit data of Claim 9, wherein the portable electronic device is selected from the group consisting of: a smart phone, a PDA, a portable game console, a digital photo frame, a notebook, a netbook, a tablet PC, and an IPAD.

16. A method for using an earphone and microphone plug to transmit biomedical data, the method comprising the steps of:
(1) inserting an earphone and microphone plug of a biomedical device into an earphone and microphone jack of a portable electronic device ;
(2) through a right channel output terminal of the earphone and microphone plug, the portable electronic device outputting a configuration data to a micro-control unit of the biomedical device;
(3) the micro-control unit receiving and analyzing the configuration data;
(4) the micro-control unit outputting a identification code to the portable electronic device via a microphone input terminal of the earphone and microphone plug;
(5) an application software installed in the portable electronic device determining the identification code;
(6) whether the identification code has been determined, if yes, proceeding to step (7), otherwise, proceeding to step (1);
(7) the communication of the micro-control unit and the portable electronic device being established;
(8) determining whether an object under test is connected to a measuring unit of the biomedical device, if yes, proceeding to step (9), otherwise, proceeding to step (8);
(9) a switch unit of the biomedical device stopping the portable electronic device outputting audio signal;
(10) determining whether the object under test can be measured, if yes, proceeding to step(11), otherwise, proceeding to step(8);
(11) the measuring unit measuring biomedical information of the object under test, and outputting the biomedical information to the micro-control unit;
(12) the biomedical information being processed to biomedical data by the micro-control unit;
(13) the micro-control unit inputting the biomedical data into the portable electronic device via the microphone input terminal;
(14) determining whether the measurement and the record of the biomedical data of the object under test is finished, if yes, proceeding to step(15), otherwise, proceeding to step(8);
(15) removing the object under test from the biomedical device; and
(16) removing the biomedical device from the portable electronic device.

17. The method for using the earphone and microphone plug to transmit the biomedical data of Claim 16, wherein the measuring unit is selected from the group consisting of: a measuring device of blood glucose strip, a measuring device of body fat, a measuring device of blood pressure, and a measuring device of electrocardiography.

18. The method for using the earphone and microphone plug to transmit the biomedical data of Claim 16, wherein the object under test is selected from the group consisting of: a blood glucose strip and a man body.

19. The method for using the earphone and microphone plug to transmit the biomedical data of Claim 16, wherein the micro-control unit inputs the biomedical data into the portable electronic device by a data frequency of 10 kHz through the microphone input terminal; moreover, the format of the biomedical data is binary code.

20. The method for using the earphone and microphone plug to transmit the biomedical data of Claim 16, wherein the portable electronic device is selected from the group consisting of: a smart phone, a PDA, a portable game console, a digital photo frame, a notebook, a netbook, a tablet PC, and an PAD.
